**Europäisches Patentamt**

(19) **European Patent Office**

**Office européen des brevets**

(11) Publication number: **0 093 138**
**B1**

(12) # EUROPEAN PATENT SPECIFICATION

(45) Date of publication of patent specification: **28.10.87**

(51) Int. Cl.⁴: **A 61 M 1/28,** A 61 M 25/02, A 61 M 1/00

(21) Application number: **82903330.7**

(22) Date of filing: **05.11.82**

(86) International application number: **PCT/GB82/00317**

(87) International publication number: **WO 83/01572 11.05.83 Gazette 83/11**

(54) **IMPROVED CONTINUOUS AMBULATORY PERITONEAL DIALYSIS SYSTEM.**

(30) Priority: **07.11.81 GB 8133663**

(43) Date of publication of application:
**09.11.83 Bulletin 83/45**

(45) Publication of the grant of the patent:
**28.10.87 Bulletin 87/44**

(84) Designated Contracting States:
**AT BE CH DE FR GB LI LU NL SE**

(56) References cited:
EP-A-0 022 370
DE-A-2 809 303
FR-A-2 390 940
US-A-3 520 298
US-A-3 633 585
US-A-4 015 601
US-A-4 184 497
US-A-4 239 041
US-A-4 256 102

(73) Proprietor: **Juhasz, Laszlo**
**Woodhead 51 Kilmardinny Avenue**
**Bearsden Glasgow G61 3NL Scotland (GB)**

(72) Inventor: **Juhasz, Laszlo**
**Woodhead 51 Kilmardinny Avenue**
**Bearsden Glasgow G61 3NL Scotland (GB)**

Courier Press, Leamington Spa, England.

### Description

The present invention relates to a dialysis system and particularly, but not exclusively, to continuous peritoneal dialysis systems for ambulatory use.

Continuous ambulatory peritoneal dialysis (CAPD) is a procedure which a patient with chronic renal failure can carry out, without hospitalisation, for example in their own home. CAPD comprises a closed loop system connecting the patient's peritoneal cavity via an indwelling catheter to a "giving set" attached to a dialysate fluid bag.

At the start of the treatment, pre-warmed peritoneal dialysis (PD) fluid is transferred from the bag into the peritoneum where it remains approximately for four hours before being drained back into the same bag. The PD-fluid bag is then changed using an aseptic technique which forms the basis of CAPD patient training. When the bag is connected, the new PD-fluid is transferred into the peritoneum by gravity flow. This is achieved by positioning the bag higher than the patient. Conversely in order to drain the peritoneum the bag is then placed below the level of the patient (peritoneum). The whole procedure then is repeated 4—5 times daily, seven days a week.

Despite an increased demand for peritoneal dialysis and related technological advances in recent years peritonitis remains the major limiting complication for CAPD therapy. The sources of infection leading to peritonitis arise principally through the lumen of the dialysis catheter and its junctions (transluminally) and along the outside of the dialysis catheter and implanted subcutaneous prosthesis (extraluminally), either producing exit-side or tunnel infections.

In addition, the present procedure is cumbersome and time-consuming; gravity PD-fluid transfer can lead to drainage (outflow) and inflow problems, junction sterilisation by fluid, such as Betadine (a proprietary name for Povidone-Iodine) can be messy and time-consuming; PD-fluid warming is frequently impractical, and outflow obstruction by omental wrap is also frequent.

In addition, although the Dacron (Trade Mark: Du Pont de Nemours, E I & Co) felt cuffs and the silicone rubber presently used are satisfactory biocompatible materials, they cannot provide an adequate skin-air interface.

Prior proposals in this field are disclosed in the following specifications:—

US 3 520 298; US 3 633 585; US 4 184 497; US 4 239 041; US 4 256 102 and EP 0 022 370, for example.

US 3 520 298 discloses a concentric tube catheter and an equal volume pump. The peritoneal nylon catheter tube thereof has a multi-junction connector located very close to the incision which can lead to increased tissue damage and infection risks every time the catheter is connected or disconnected.

US 3 633 585 discloses a catheter requiring a skirt of Dacron or Teflon which has apertures allowing tissue invasion and a similar sleeve which is sutured to the inner face of the peritoneum. Without the sleeve and sutures, desirable free movement of the catheter is possible. This catheter also has a junction which is very close to the incision giving rise to problems of tissue damage and infection arising from manipulation of the catheter to effect connection and disconnection.

US 4 184 497 provides a catheter having porous cuff means and a collar for initial suturing of the catheter to the abdominal wall. An implant of a needle-permeable material of a hollow construction is required for this catheter. After healing of the incisions required for inserting the implant, peritoneal dialysis is accomplished by puncturing the skin above the implant, and the implant itself with a peritoneal dialysis solution needle. US 4 256 102 provides a similar method of dialysis using a differently shaped implant and a catheter tube secured by means of a Dacron cuff to the fascia.

US 4 239 041 which is equivalent to DE 2 809 303 discloses a gravity flow system utilizing a catheter located by means of a Dacron felt cuff which as mentioned above has not proved entirely satisfactory in providing an adequate skin-air interface.

EP 0 022 370 discloses a two-part catheter having a retainer section which is permanently located in the abdominal wall by tissue growth into a felt cuff portion, and a releasable section coupled through a fluid-tight seal for normal use, the said seal being normally located within the retainer section in the abdominal wall. The retainer section is formed, for example, from a soft silicone rubber sleeve and a porous Dacron felt ring.

An object of the present invention is to obviate or mitigate the abovesaid disadvantages.

According to the present invention there is provided a peritoneal dialysis system comprising a catheter having a portion insertable into the peritoneum, the catheter being connectable to a dialysis fluid chamber by a single connection, means for controlling the flow of dialysis fluid from said chamber, and catheter securing means firmly attached to said catheter for connecting said catheter to tissue, characterised in that said securing means is composed of activated charcoal impregnated with uncrosslinked phenolic resin.

Preferably, said catheter securing means comprises two cuff portions, one cuff portion for securing the catheter to the skin surface, the other cuff portion for securing the catheter to the fascia. Alternatively, said catheter securing means may extend continuously along the catheter from the skin surface to the fascia.

Preferably also, the catheter has a curved portion, the curved portion having a plurality of apertures therein.

Preferably also, the means for controlling the flow is a non-fluid contacting pump, said pump being manually actuatable by the patient.

Preferably also, the means for controlling the flow of dialysis fluid includes a fluid switch located on-line in the catheter.

Preferably also, the uncrosslinked resin within the charcoal-resin composite is heated to a temperature just below the onset of carbonisation.

Preferably also, the heat treatment is conducted in nitrogen, the temperature being cycled between ambient and 350°C at a constant rate. Preferably the constant rate is 0.1°C/min.

An embodiment of the present invention will now be described by way of example with reference to the accompanying drawings in which:—

Fig. 1 is a schematic diagram of a catheter in accordance with the invention located in the peritoneum;

Fig. 2 is a diagram of the attachment of the catheter to the permanent "giving set";

Fig. 3 shows a complete continuous ambulatory peritoneal dialysis system in accordance with the present invention.

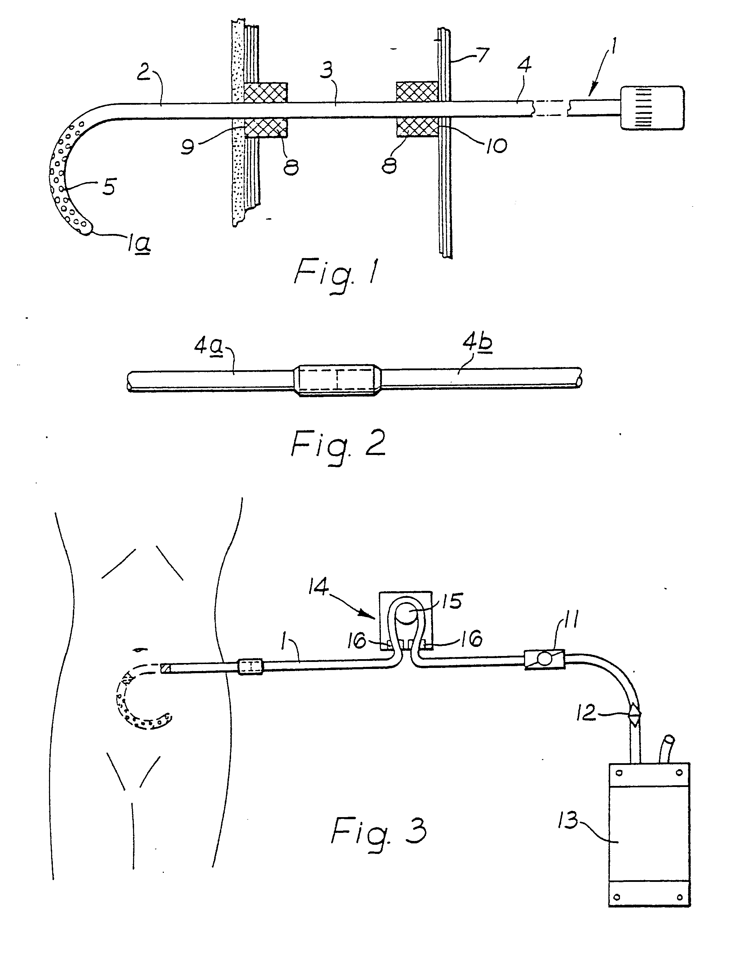

Referring now to Fig. 1, the catheter has three well defined sections; intraperitoneal 2, subcutaneous 3, and external 4. It has an overall diameter of 5 mm and a bore size of 3 mm, providing 34% greater free volume flow than conventional catheters.

The intraperitoneal section 2 of the PD catheter 1 is about 160 mm long and is perforated for about 100 mm of its length with 48 spaced 0.6 mm holes 5. The tip 1a of the catheter 1 is rounded off to avoid intraperitoneal irritation. The full length of section 2 lies in a curved form in the peritoneal cavity in such a way as to minimise omentum or bowel wrapping.

The subcutaneous section 3 is about 90 mm long and starts just outside of the peritoneum and extending up to the epidermis 6 (5—10) mm below the skin surface 7. Outside of this section soft porous carbon cuffs 8 or alternatively a full length sleeve (not shown) are glued on with medical grade silastic adhesive.

The carbon cuff is fabricated from activated charcoal tubing impregnated with phenolic resin (about 10—15% by volume). One suitable resin is Type J20/317A (TM) produced by B.P. Chemicals. Any suitable conventional activated charcoal tubing may be used, but preferably the weave should be fairly open. The uncrosslinked polymer within the porous charcoal composite is heated to a temperature below that of onset of carbonisation. In this way the flexibility of the base material can be maintained where the excess volatile constituents are eliminated.

The heat treatment is conducted in an inert nitrogen atmosphere and involves a controlled temperature cycle between ambient and 350°C at a rate of 0.1°C/min. The upper limit of temperature is about 750°C about which the charcoal carbonises and becomes inflexible and the upper rate is about 0.5°C/min above which the quality of the composite is degraded by 'bubbling' for example. If the cycling rate is too fast the solvent cannot escape and retention of the solvent alters the mechanical properties of the composite and makes it less flexible and brittle. The flexible carbon subcutaneous tissue interface due to its porous structure and inherent biocompatibility encourages viable tissue ingrowth and thus minimises tunnel infection. It also provides better physiological and mechanical sealing at the peritoneum interface 9 and at the epidermis interface 10.

The extra-abdominal section 4 of the catheter 1 has a continuous length of 2 m. It is made up of two parts, one is the external section of the implanted catheter 4a and the other is a permanent "giving set" 4b. After 48 hours of implantation the two sections are permanently glued together with overlaying silicone rubber sleeving and adhesive as illustrated in Fig. 2. This two part arrangement facilitates implantation and also if it becomes necessary, repositioning of the catheter in the event of clinical complications.

The use of a permanently extended catheter eliminates the need to change the giving set and provides a single junction closed loop CAPD system and is also more cost effective. This design firstly minimises the number of contamination sites for possible infection and also contributes to a reduction in running cost for CAPD therapy.

The extra-abdominal silicone rubber tubing also has an on-line fluid switch 11 and durable polycarbonate-stainless steel (PS) junction 12 (Fig. 3). This single PS junction connects the catheter 1 to the flexible PD bag 13. A roller pump 14 receives the catheter 1, the catheter being located round the roller 15. The catheter is held in place by flanges 16. The pump 14 is driven by a small d.c. electric motor (not shown), the speed of which may be varied. The motor is powered by rechargeable batteries which may be recharged from the same 12v d.c. supply used to heat the dialysis fluid. This unit is small, portable and there is no contact between the dialysis fluid and the roller 15 which reduces the possibility of contamination. The whole assembly is sterilisable, for example, by autoclaving and may be used as a permanent fixture by the patient. Polycarbonate may be replaced by any other suitable sterilisable material. The PS junction 12 is a double helix luer-type design which permits a single twist-on twist-off connection to the PD fluid bag 13. This ensures a secure leakproof union during fluid transfer. The outside stainless steel body has a functional grip section and inside a polycarbonate luer-type cone which cannot be touch contaminated. Thus the system consists of an extended long silicone rubber catheter which has a carbon subcutaneous section and a single PS junction connection between the peritoneum and the PD fluid bag.

Advantages of the system are that:

The roller pump system enables the patient to perform dialysis at any location without the requirement of being restricted to finding suitable positions for the dialysis bag, this also means that the patient may be mobile during dialysing.

When using the roller pump it is convenient to measure the electrical conductivity of the fluid inflow and outflow at the fluid junction which will provide a rapid indication of the effectiveness of the dialysis.

It also uses as a permanent accessory an on-line fluid switch for safety and convenience and the resin impregnated carbon cuffs provide a good biocompatible interface between the skin and air; and the use of a soft carbon tissue interface coupled with a single junction design considerably lowers the probability of the rate of infection and of peritonitis episodes.

## Claims

1. A peritoneal dialysis system comprising a catheter (1) having a portion (2) insertable into the peritoneum, the catheter (1) being connectable to a dialysis fluid chamber (13) by a single connection (12), means (11) for controlling the flow of dialysis fluid from said chamber (13), and catheter securing means (8) firmly attached to said catheter (1) for connecting said catheter (1) to tissue, characterised in that said securing means (8) is composed of activated charcoal impregnated with uncrosslinked phenolic resin.

2. The system of claim 1, in which the charcoal-resin composite (8) has been heat-treated to a temperature below the onset of carbonisation.

3. The system of claim 2, in which the heat-treatment is conducted in a nitrogen atmosphere, the temperature being cycled between ambient and 350°C at a constant rate.

4. The system of claim 1 in which said catheter securing means (8) comprises two cuff portions (8), one cuff portion (8) for securing the catheter (1) to the skin surface (10), the other cuff portion (8) for securing the catheter (1) to the fascia (9).

5. The system of claim 1 in which said catheter securing means (8) is a sleeve extending continuously along the catheter (1) a length equivalent to the distance between the skin surface (10) and the fascia (9).

6. The peritoneal dialysis system of any one of claims 1 to 5 characterized in that the catheter (1) has an extra-abdominal section (4) made up of two parts to form a permanently extended catheter, the first of said parts being the external section (4a) of the implanted catheter (1) and the other being a permanent 'giving set' (4b), the said parts being permanently securable together.

7. The system of claim 6 wherein the said parts (4a) and (4b) are permanently securable together with overlaying silicon rubber sleeving and adhesive.

8. The system of claim 6 or claim 7 in which the part (4b) comprises an extended long silicone rubber tubing having at one end thereof a stainless steel/polycarbonate junction (12) for attaching said tubing to a fluid bag (13).

9. The system of claim 8 in which the said junction (12) is a double helix luer-type design which permits a single twist-on, twist-off connection to the fluid bag (13).

10. The system of claim 6 in which the extra-abdominal section (4) of the catheter (1) has a continuous length of 2 m.

11. The system of any preceding claim in which the catheter (1) has a curved portion (2), the curved portion (2) having a plurality of apertures (5) therein.

12. The system of any preceding claim in which the means for controlling the flow of dialysis fluid is a non-fluid contacting roller pump (14), said pump being manually actuatable by the patient.

13. The system of any preceding claim in which the means for controlling the flow of dialysis fluid includes a fluid switch (11) located on-line in the catheter (1).

14. The system of any preceding claim in which the catheter (1) has an overall diameter of 5 mm and a bore size of 3 mm.

## Patentansprüche

1. System für peritoneale Dialyse, bestehend aus einem Katheter (1) mit einem in das Peritoneum einführbaren Abschnitt (2), wobei der Katheter (1) mit Hilfe einer Einzelverbindung (12), einer Vorrichtung (11) zur Kontrollierung des Flusses der Dialyseflüssigkeit aus der Dialyseflüssigkeitskammer (13) und einer zur Verbindung des Katheters (1) mit dem Gewebe an dem Katheter (1) fest angebrachten Sicherungsvorrichtung (8) an die Flüssigkeitskammer (13) angeschlossen werden kann, dadurch gekennzeichnet, dass die Sicherungsvorrichtung (8) aus mit nicht vernetztem Phenolharz imprägnierter Aktivkohle besteht.

2. System nach Anspruch 1, dadurch gekennzeichnet, dass das Harz-Kohle-Element (8) mit einer Temperatur unterhalb der Karbonisationsgrenze wärmebehandelt wurde.

3. System nach Anspruch 2, dadurch gekennzeichnet, dass die Wärmebehandlung in einer Stickstoffatmosphäre durchgeführt wird, wobei die Temperatur sich in einem konstanten Zyklus zwischen der Umgebungstemperatur und 350°C bewegt.

4. System nach Anspruch 1, dadurch gekennzeichnet, dass die Sicherungsvorrichtung (8) des Katheters aus zwei Umschliessungselementen (8) besteht, wobei das eine Umschliessungselement (8) zur Befestigung des Katheters (1) an der Hautoberfläche (10) und das andere Umschliessungselement (8) zur Befestigung des Katheters (1) an der Frontfläche (9) dienen.

5. System nach Anspruch 1, dadurch gekennzeichnet, dass die Sicherungsvorrichtung (8) des Katheters eine sich auf einer dem Abstand zwischen der Hautoberfläche (10) und der Frontfläche (9) entsprechenden Strecke gleichmässig entlang des Katheters verlaufende Ummantelung darstellt.

6. System für peritoneale Dialyse nach einem der Ansprüche 1 bis 5, dadurch gekennzeichnet, dass der Katheter (1) einen extraabdominalen Abschnitt (4) aufweist, welcher einen aus zwei Teilen bestehenden permanent erweiterten Katheter bildet, wobei das erste dieser Teile der äussere Abschnitt (4a) des implantierten Katheters (1) ist und das andere Teil eine permanente "Zuführung" (4b) darstellt und beide Teile permanent miteinander verbunden werden können.

7. System nach Anspruch 6, dadurch gekennzeichnet, dass die Teile (4a) und (4b) durch Überlagerungen aus einem Silikonkautschukmantel und Haftstoff permanent miteinander verbunden werden können.

8. System nach Anspruch 6 oder Anspruch 7, dadurch gekennzeichnet, dass die Teil (4b) aus einer erweiterten länglichen Röhre aus Silikonkautschuk besteht, welche an einem ihrer Enden ein Verbindungsstück (12) aus nichtrostendem Stahl/Polykarbonat zur Verbindung der Röhre mit einem Flüssigkeitsbehälter (13) aufweist.

9. System nach Anspruch 8, dadurch gekennzeichnet, dass das Verbindungsstück (12) eine Luer-artige Doppelspirale darstellt, welche eine einzelne Auf-Zu-Drehverbindung mit dem Flüssigkeitsbehälter zulässt.

10. System nach Anspruch 6, dadurch gekennzeichnet, dass der extraabdominale Abschnitt (4) des Katheters (1) eine gleichmässige Länge von 2 m aufweist.

11. System nach einem der vorhergehenden Ansprüche, dadurch gekennzeichnet, dass der Katheter (1) einen gekrümmten Abschnitt (2) hat, wobei der gekrümmte Abschnitt (2) eine Vielzahl von Öffnungen (5) aufweist.

12. System nach einem der vorhergehenden Ansprüche, dadurch gekennzeichnet, dass die Vorrichtung zur Kontrollierung des Flusses der Dialyseflüssigkeit eine Rollenpumpe (14) ist, welche nicht mit der Flüssigkeit in Berührung kommt, wobei die Pumpe vom Patienten manuell betätigt werden kann.

13. System nach einem der vorhergehenden Ansprüche, dadurch gekennzeichnet, dass die Vorrichtung zur Kontrollierung des Flusses der Dialyseflüssigkeit einen direkt am Katheter (1) angebrachten Flüssigkeitsschalter (11) aufweist.

14. System nach einem der vorhergehenden Ansprüche, dadurch gekennzeichnet, dass der Katheter (1) einen Gesamtdurchmesser von 5 mm und einen inneren Durchmesser von 3 mm aufweist.

**Revendications**

1. Un système de dialyse péritonéale comprenant un cathéter (1) dont une portion (2) peut s'insérer dans le péritoine, le cathéter (1) pouvant être relié à une poche de liquide de dialyse (13) par un joint simple (12), un dispositif (11) de contrôle du débit du liquide de dialyse de la dite poche (13), et un dispositif de fixation du cathéter (8) solidement fixé sur le dit cathéter (1) pour joindre le dit cathéter (1) au tissu, caractérisé en ce que le dit dispositif de fixation (8) est composé de charbon actif imprégné de résine phénolique réticulée.

2. Le système selon la revendication 1, dans lequel le composé charbon-résine (8) a été traité a la chaleur jusqu'à une température inférieure au point de carbonisation.

3. Le système selon la revendication 2, dans lequel le traitement par la chaleur est effectué dans une atmosphère de nitrogène, la température suivant un cycle entre la température ambiante et 350°C à un taux constant.

4. Le système selon la revendication 1 dans lequel le dit dispositif de fixation du cathéter (8) comprend deux parties manchons (8), une partie manchon (8) pour fixer le cathéter (1) à la surface de la peau (10), l'autre partie manchon pour fixer le cathéter (1) sur le fascia (9).

5. Le système selon la revendication 1 dans lequel le dit dispositif de fixation du cathéter (8) est une gaine s'étendant de manière continue le long du cathéter (1) sur une longueur équivalente à la distance entre la surface de la peau (10) et le fascia (9).

6. Le système de dialyse péritonéale selon l'une des revendications 1 à 5 caractérisé en ce que le cathéter (1) a une section extra-abdominale (4) constituée de deux parties de manière à former un cathéter allongé en permanence, la première des dites parties étant la section externe (4a) du cathéter implanté (1) et l'autre étant un "élément donateur" permanent (4b), les dites parties étant en permanence reliables entre elles.

7. Le système selon la revendication 6 où les dites parties (4a et 4b) sont en permanence reliables entre elles si on les recouvre d'une gaine de caoutchouc silicone avec de l'adhésif.

8. Le système selon la revendication 6 ou selon la revendication 7 dans lequel la partie (4b) comprend un long tube allongé de caoutchouc silicone ayant en une extrémité un raccord d'acier inoxydable/polycarbonate (12) pour attacher le dit tube à une poche à fluide (13).

9. Le système selon la revendication 8 dans lequel le dit raccord (12) est du type luer à double hélice qui permet une connexion simple par vissage-dévissage à la poche à liquide (13).

10. Le système selon la revendication 6 dans lequel la section extra-abdominale (4) du cathéter (1) a une longueur continue de 2 m.

11. Le système de l'une des revendications précédentes dans lequel le cathéter (1) a une portion recourbée (2), la portion recourbée (2) ayant une pluralité d'ouvertures (5).

12. Le système selon l'une des revendications précédentes dans lequel le dispositif de contrôle du débit du liquide de dialyse est une pompe à rouleaux sans contact avec le fluide (14), la dite pompe pourant être commandée manuellement par le patient.

13. Le système selon l'une des revendications précédentes dans lequel le dispositif de contrôle du débit du liquide de dialyse comprend un interrupteur de fluide (11) situé le long du cathéter (1).

14. Le système selon l'une des revendications précédentes dans lequel le cathéter (1) a un diamètre total de 5 mm et un calibre de 3 mm.

*Fig. 1*

*Fig. 2*

*Fig. 3*